# EUROPEAN PATENT APPLICATION

(11) **EP 4 403 095 A1**
(43) Date of publication of application: **24.07.2024**
(21) Application number: 23152101.4
(22) Date of filing: 17.01.2023
(51) Int. Cl.: A61B 5/00, A61B 5/145

(54) **PROCESS FOR DETECTING ACOUSTIC LEAKS IN A NON-INVASIVE SENSOR BASED ON INDIRECT PHOTOACOUSTIC DETECTION**

(71) Applicant: Eclypia, 38000 GRENOBLE (FR)
(72) Inventor: COUTARD, Jean-Guillaume, 38660 Plateau-des-Petites-Roches (FR); MONPEURT, Cyrielle, 38000 GRENOBLE (FR); JOBERT, Daphnée, 38000 GRENOBLE (FR); MATHIEU, Grégoire, 38000 GRENOBLE (FR); BLANC, Romain, 38000 GRENOBLE (FR)
(74) Representative: Fidal Innovation

(57) **Abstract**

A process for detecting acoustic leaks in a non-invasive sensor based on indirect photoacoustic detection, comprising:
a) measuring with a detection cell a plurality of amplitudes and/or phases of pressure waves generated in the detection cell in response to a plurality of irradiations of a test target with at least one light source, the intensity of the emitted light being modulated at a modulation frequency that is different for each of the plurality of irradiations;
b) Determining with a processor at least one acoustic response of the detection cell as a function of a frequency of a pressure wave, based on the plurality of amplitudes and/or phases of pressure waves measured at a) and the respective modulation frequencies.
c) Comparing with a processor at least one determined acoustic response of the detection cell to at least one reference acoustic response;
d) Based on the result of the comparison, providing with a processor a detection result characterizing acoustic leaks in the detection cell.

## Description

### FIELD OF THE INVENTION

The present invention relates to a photoacoustic sensor.

More specifically, the invention relates to a process for detecting acoustic leaks in a sensor based on photoacoustic detection, the sensor comprising an acoustic transducer and being configured for measuring a parameter of interest in a target medium and being, for example, a non-invasive glucose sensor based on indirect photoacoustic detection.

### TECHNOLOGICAL BACKGROUND

In the field of sensors for living organisms, non-invasive sensors based on photoacoustic or photothermal detection are known.

In such sensors, an area of interest of a medium to be analyzed, also called target, is irradiated by means of a laser beam of wavelength(s) and modulation frequency(ies) chosen according to the parameter of interest to be measured.

The laser beam is absorbed by the target over a characteristic length that depends on the structure of the target. The absorption of light energy leads to local heating of the target. In response to this heating, a thermal wave having a frequency equal to the modulation frequency of the laser is generated in the target. This thermal wave propagates in the target and, in particular, up to the outer surface of the target.

The thermal wave can be directly detected and analyzed. In this case, we speak of photothermal detection.

Photoacoustic detection exploits the fact that the thermal wave is associated with a pressure wave having a frequency identical to the modulation frequency of the laser irradiating the target. In the case of "indirect photoacoustic detection", the photoacoustic sensor detects the pressure wave generated in the fluid external environment after propagation of the thermal wave through the target up to the interface between the target and the fluid external environment.

The photoacoustic effect has been the subject of numerous theoretical studies. Allan Rosencwaig and Allen Gersho have developed a theoretical model of the photoacoustic signal. This model involves the physico-chemical properties of the sample to be analyzed, including the optical scattering length, the thickness and the thermal scattering length of the sample. (Rosencwaig, A. and Gersho, A. (1976), Theory of the photoacoustic effect with solids, *Journal of Applied Physics,* 47, 64).

Hu et al. developed a generalized theory of the photoacoustic effect in a laminated material. (Hu, H., Wang, X., & Xu, X. (1999). Generalized theory of the photoacoustic effect in a multilayer material. Journal of Applied Physics, 86, 3953-3958.)

Photoacoustic detection has many advantages over other detection techniques, including the orthogonal aspect of transduction: the optical signal at the input of the medium to be analyzed is converted into an acoustic signal :
- that is very specific to the phenomenon to be observed,
- and that allows the use of inexpensive and miniaturized sensors.

The difficulty of photoacoustic detection stems, among other things:
- from the number of parameters generally influencing the detected signal and,
- for certain analytes of interest present at low concentration in the medium to be analyzed, from the small proportion of the detected signal specific to each of these parameters of interest.
Photoacoustic detection requires not only a choice of wavelength(s) as a function of the parameter of interest but also a choice of the modulation frequency(ies) of the laser to be used. Indeed, the characteristic length of penetration of the incident light wave into the target depends on the modulation frequency of the laser and the component to be detected can be located more or less deeply under the external surface of the target.

Whatever the wavelength and the modulation frequency chosen for the irradiation of the target, a thermal wave is generated in the target, that gives rise in the environment of the target to an associated pressure wave (or equivalently acoustic wave) having a frequency that is equal to the modulation frequency of the irradiation light.

This acoustic wave can be detected with an acoustic detection cell, such as the ones described in EP3885765. More particularly, usual acoustic detection cells comprise a detection chamber delimited by walls and filled with gas, such as air, and an acoustic transducer, such as, by way of example, a microphone.

The amplitude of the acoustic signal sensed by the acoustic transducer depends on the intrinsic characteristics of the acoustic transducer but also on the characteristics of the detection chamber, such as its volume and the gas filling the detection chamber.

EP3885765 clearly shows that the amplitude of the sensed acoustic signal also highly depends on the openings in the walls of the detection chamber. In particular, EP3885765 shows that, if the detection chamber is in contact with ambient air at atmospheric pressure through a channel, the detection chamber can act - in the range of frequencies tested - either as a low pass filter without resonance frequency or as a band pass filter with a resonance frequency depending on the diameter of the channel. The applicants associate these two limit responses with the fact that the chamber can be considered as closed or open depending on the diameter of the channel. As a consequence, EP3885765 proposes optimal ranges for the channel diameter associated with particular optimal ranges for the chamber volume, in order to increase the amplitudes of the acoustic waves received by the acoustic transducer.

However, if EP3885765 proposes an optimal choice for the geometric parameters of a detection chamber at the design stage, the inventors of the present application have observed that during the lifetime of a detection cell, the detection chamber doesn't always behave as expected. In particular, a detection chamber designed to behave as an acoustic low pass filter sometimes behaves as an acoustic pass band filter, which could be associated with unwished acoustic leaks. These acoustic leaks can have a significant impact on the optimality of the geometric parameters of the detection chamber regarding the amplitude of the detected acoustic waves, and therefore, on the precision of the detection.

Since the detection cell of a non-invasive sensor based on indirect photoacoustic doesn't always behave as expected during its lifetime, the invention aims at proposing a process for increasing the confidence level in the acoustic behavior of the detection cell of a non-invasive sensor based on indirect photoacoustic detection, among others in view of a subsequent measurement of a parameter of interest.

### SUMMARY OF THE INVENTION

Hence, the invention relates to a process for detecting acoustic leaks in a non-invasive sensor based on indirect photoacoustic detection and configured for measuring at least one parameter of interest in a target, wherein said process comprises:
a) measuring with a detection cell of the non-invasive sensor a plurality of amplitudes and/or phases of pressure waves generated in the detection cell in response to a plurality of irradiations of a test target with at least one light source emitting a light characterized by at least one wavelength, the intensity of the respective emitted light being modulated at a modulation frequency that is different for each of the plurality of irradiations;
b) Determining with a processor at least one acoustic response of the detection cell of the non-invasive sensor as a function of a frequency of a pressure wave, based on the plurality of amplitudes and/or phases of pressure waves measured at a) and the respective modulation frequencies.
c) Comparing with a processor at least one determined acoustic response of the detection cell to at least one reference acoustic response;
d) Based on the result of the comparison, providing with a processor a detection result characterizing acoustic leaks in the detection cell.

Such a process allows to compare a determined (and thus a current) acoustic response of the detection cell of a non-invasive sensor based on indirect photoacoustic detection and a reference acoustic response. The reference acoustic response can be specific to the detection cell that is made use of or associated with a range or a type of detection cell. The reference acoustic response is associated with an expected behaviour of the detection cell. Thus, the result of the comparison between the determined acoustic response and the reference response is representative of the fact that the detection cell behaves as expected or not, and as a consequence, of the fact that acoustic leaks could be associated or not with the detection cell at the time the process is performed. Hence, the process according to the invention allows to increase the confidence level in the acoustic behaviour of the detection cell at a given time point and in a subsequent measurement of a parameter of interest with the non-invasive sensor based on indirect photoacoustic detection.

In a particular embodiment of the process for detecting acoustic leaks in a non-invasive sensor based on indirect photoacoustic detection, at a), a plurality of acoustic responses of the detection cell are determined with a plurality of acoustic transducers and/or at a plurality of irradiation wavelengths, and at b) a plurality of determined acoustic responses is compared to a plurality of reference acoustic responses.

The use of a plurality of acoustic response determined with a plurality of acoustic transducers can reinforce the confidence level in the result of the detection of acoustic leaks.

In a particular embodiment of the process for detecting acoustic leaks in a non-invasive sensor based on indirect photoacoustic detection, the comparison is based on a comparison metric involving at least one metric variable chosen among a number of resonance frequencies of the acoustic response of the detection cell,at least one sense of variation of the amplitude and/or the phase of the pressure wave generated in the detection cell as a function of the frequency in at least one predetermined frequency range, a spectral angle mapper score and a root-mean-square deviation.

In a particular embodiment of the process for detecting acoustic leaks in a non-invasive sensor based on indirect photoacoustic detection, the detection result is a discrete variable.

In this case, the user can associate the discrete variable with a discrete level of leaks, such as for example leaks / no leaks, or no leaks / small leaks / moderate leaks/ important leaks, and decide on a further action based on this result.

In a particular embodiment of the process for detecting acoustic leaks in a non-invasive sensor based on indirect photoacoustic detection, the detection result is a continuous variable.

In a particular embodiment of the process for detecting acoustic leaks in a non-invasive sensor based on indirect photoacoustic detection, the process comprises after d):
e) if the detection result is lower than a predetermined threshold, allowing the non-invasive sensor to perform at least one subsequent measurement of the parameter of interest in a main target and if the detection result is higher than a predetermined threshold, transmitting to a user of the non-invasive sensor at least one instruction to remedy the detected acoustic leak. This embodiment allows :
- either a validation of the non-invasive sensor for a subsequent measurement in a main target if no leaks or small enough leaks (that do not significantly impare the non-invasive sensor) are detected,
- or the user to take appropriate actions if important leaks are detected.

In a particular embodiment of the process for detecting acoustic leaks in a non-invasive sensor based on indirect photoacoustic detection, the at least one instruction transmitted to the user is to check the position of the non-invasive sensor relative to the test target.

Since the correct positioning of the non-invasive sensor relative to the test target is a major parameter of the measurement process and that the position of the sensor can in many cases involuntarily evolve, such an instruction allows to eliminate a frequent cause of acoustic leaks. In a particular embodiment of the process for detecting acoustic leaks in a non-invasive sensor based on indirect photoacoustic detection, if the user determines that the position of the non-invasive sensor relative to the test target is correct, a user of the non-invasive sensor is further instructed to disqualify the non-invasive sensor for subsequent measurements and optionally to replace the non-invasive sensor.

Such an embodiment allows to disqualify the non-invasive sensor only if the detected acoustic leaks cannot be associated with an incorrect position of the non-invasive sensor relative to the test target.

The invention also relates to a process for measuring at least one parameter of interest with a non-invasive sensor based on indirect photoacoustic detection and configured for measuring at least one parameter of interest in a main target, the process comprising carrying out the process according comprising a step e) described above on a non-invasive sensor with a test target, and if the non-invasive sensor is allowed at e) to perform at least one subsequent measurement, performing at least on subsequent measurement of the parameter of interest in said main target. Such a measuring process allows to reinforce the confidence level in the measurement process, since the measurement is performed only once the absence of acoustic leaks in the detection cell has been confirmed.

In a particular embodiment of the process for measuring at least one parameter of interest with a non-invasive sensor based on indirect photoacoustic detection, the test target for the process for detecting acoustic leaks is also the main target for a subsequent measurement of at least one parameter of interest with said non-invasive sensor.

Such an embodiment allows in situ detection of acoustic leaks, and if repeated, continuous monitoring both of the acoustic leaks in the detection cell and the at least one parameter of interest with a higher confidence level thanks to the monitoring of the acoustic leaks.

In a particular embodiment of the process for measuring at least one parameter of interest with a non-invasive sensor based on indirect photoacoustic detection, the test target for the process for detecting acoustic leaks is different from the main target for a subsequent measurement of at least one parameter of interest with said non-invasive sensor.

Such an embodiment is more particularly adapted for testing, and optionally qualifying a non-invasive sensor at the end of a production line.

The invention further relates to a computer program comprising instructions which, when the program is executed by a processor, cause the processor to carry out step b), step c) and step d) of the process according to claim 1.

Last, the invention deals with a non-invasive sensor based on indirect photoacoustic detection comprising a detection cell, at least one light source emitting a light characterized by at least one wavelength, the intensity of the respective emitted light being modulated at a modulation frequency that is tunable, and a processor configured for:
- determining at least one acoustic response of the detection cell of the non-invasive sensor as a function of a frequency of a pressure wave, based on a plurality of amplitudes and/or phases of pressure waves measured with the detection cell;
- comparing at least one determined acoustic response of the detection cell to at least one reference acoustic response; and,
- based on the result of the comparison, providing a detection result characterizing acoustic leaks in the detection cell.

### BRIEF DESCRIPTION OF THE DRAWINGS

Embodiments of the invention will be described below, in relation to the following drawings :
**Figure 1** shows an exemplary embodiment of a photoacoustic sensor suitable for carrying out the process according to the invention.
**Figure 2** shows an exemplary embodiment of a photoacoustic detection cell of a photoacoustic sensor suitable for carrying out the process according to the invention optimally positioned relative to a target.
**Figure 3** is a reproduction of figure 4C of EP3885765. This graph represents the response in terms of average amplitude of the pressure generated in a detection chamber having a volume equal to 5 µL and connected to the ambient air through a tube, the length of the tube being 9.4 mm, for various tube diameters, equal to 110 µm, 220 µm and 360 µm, respectively, for modulation frequencies of the irradiation light ranging from 10² Hz to 10⁴ Hz.
**Figure 4** represents the same photoacoustic detection cell of a photoacoustic sensor as in figure 2 but not optimally positioned relative to the target.
**Figure 5** represents the same photoacoustic detection cell of a photoacoustic sensor as in figure 2 in which an acoustic transducer has partially unsealed.
**Figure 6a** (respectively **Figure 6b****)** shows the simulated acoustic response in terms of amplitude (respectively phase) of the pressure wave of a model of detection chamber of a photoacoustic sensor, the simulation being performed with COMSOL Multiphysics^{®}, for several diameters of a hole situated near the microphone and simulating acoustic leaks (no hole, diameter of 50 µm, 75 µm, 100 µm, 150 µm).
**Figure 6c** (respectively **Figure 6d****)** shows the simulated acoustic response in terms of amplitude (respectively phase) of the pressure wave of a model of detection chamber of a photoacoustic sensor, the simulation being performed with COMSOL Multiphysics^{®}, for several diameters of holes situated near the target (skin model) and simulating acoustic leaks (no hole, diameter of 50 µm, 75 µm, 100 µm, 150 µm).
**Figure 7** is an exemplary embodiment of the process for detecting acoustic leaks according to the invention.
**Figure 8** shows the acoustic responses of the detection chamber of a real first non-invasive glucose sensor determined in the same conditions but with three different light sources 11a : QCL1, having a wavenumber of 1034 cm⁻¹, QCL2, having a wavenumber of 950 cm⁻¹, and QCL3, having a wavenumber of 1125 cm⁻¹.
**Figure 9** shows the acoustic responses of the detection chamber of a second real non-invasive glucose sensor determined in the same conditions but with three different light sources 11a : QCL1, having a wavenumber of 1034 cm⁻¹, QCL2, having a wavenumber of 950 cm⁻¹, and QCL3, having a wavenumber of 1125 cm⁻¹

On the drawings, the same reference signs show the same or similar objects.

### DETAILED DESCRIPTION

The invention relates to a process to be implemented on a non-invasive sensor 1 configured for analyzing one or more parameters of a target 2, the structure of which possibly evolves over time. The target 2 may be, for example, a tissue of a human or animal organism, such as skin. The one or more parameters to be measured are called hereafter "parameters of interest".

More specifically, a parameter of interest may be a physiological parameter in the case where the target 2 is a tissue of a human being or an animal. For example, a physiological parameter to be measured is blood glucose, and more particularly interstitial blood glucose.

In other exemplary embodiments of the non-invasive sensor 1, a physiological parameter to be measured can also be the water content or the lactate concentration of a particular layer of the skin.

The non-invasive sensor 1 can be portable, and/or it can allow continuous monitoring of the parameter(s) of interest.

The non-invasive sensor 1 is based on indirect photoacoustic detection.

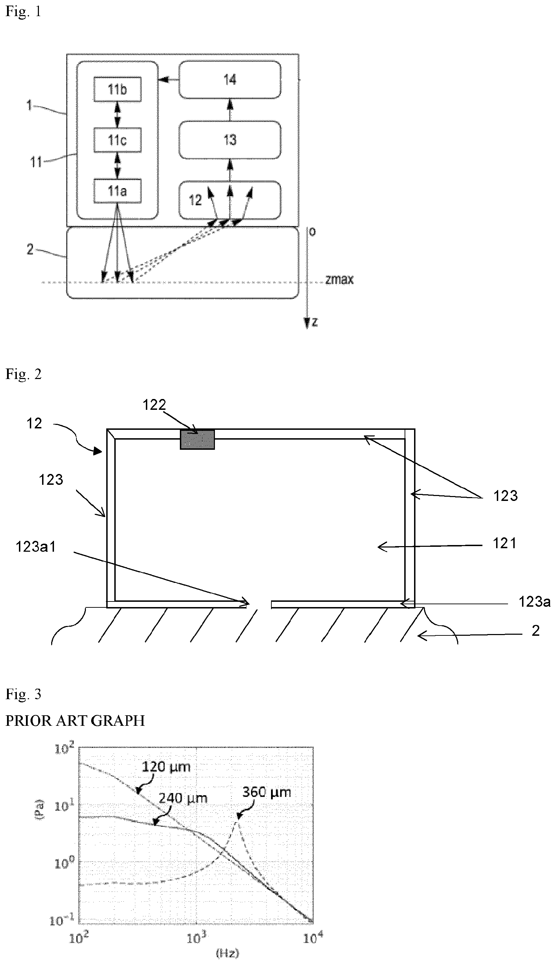

Figure 1 is a schematic of an exemplary non-invasive sensor 1 based on indirect photoacoustic detection suitable for implementing the process according to the invention. In Figure 1, the non-invasive sensor 1 comprises:
- an irradiation device 11 comprising:
   * a light source 11a,
   * a device for modulating the intensity of this light source 1 1b,
   * a control device 11c of at least one modulation frequency at which the intensity modulation device 1 1b modulates the intensity of a light emitted by the light source 1 1a;
- at least one photoacoustic detection cell 12 configured for detecting an acoustic signal generated in response to an irradiation of a target 2 with the irradiation device 11, the acoustic signal being correlated with a thermal wave that propagates in a target 2;
- a signal processing module 13 configured to receive and process data from the at least one photoacoustic detection cell 12;
- an adaptation module 14 for adapting the irradiation parameters of the irradiation device 11. All the elements of the non-invasive sensor 1 can be embedded in the same housing or part of these elements, in particular the adaptation module 14, can be remote so that the non-invasive sensor 1 forms a distributed environment.

In the case where parts of the elements of the non-invasive sensor 1 are remote, the non-invasive sensor 1 comprises means of communication enabling the required data exchanges.

### Light source 11a

In a particular embodiment, the light source 11a emits an intensity modulated laser beam at at least one wavelength λ towards the target 2.

The at least one wavelength can be chosen according to one of the parameters of interest. For example, a wavenumber equal to 1034 cm⁻¹, corresponding to a maximum of absorption of glucose, may be relevant if the non-invasive sensor 1 is a blood glucose sensor.

The light source 11a can be a light-emitting diode (LED) or a laser device. The light source 11a can, in addition or in variant, include a quantum cascade laser (QCL) emitting in the mid-infrared region (MIR-QCL), an Interband Cavity Laser (ICL), a laser having an internal or external cavity, a GaSb laser. These examples are not limiting.

The light source 11a and its irradiation parameters (wavelength(s), modulation frequency(ies), power) can be chosen according to the target 2 and/ or to one or more parameters of interest. The non-invasive sensor 1 can include several different and/or identical light sources 11a.

The non-invasive sensor 1 also includes the circuitry associated with the light source(s) 11a and at least one control device 11c configured to control the frequency at which at least one intensity modulation device 1 1b modulates the intensity of at least one light source 1 1a, so that the intensity of the light emitted by the light source 11a is modulated at an adjustable modulation frequency.

A modulation frequency of the intensity of the light emitted by a given light source 11a at a particular wavelength λ is called hereafter fmod. Several modulation frequencies fmod can be associated with the same wavelength and several wavelengths can be associated with (or equivalently correspond to) the same modulation frequency such that the light emitted by a given light source 11a can be described in terms of pairs of characterizing parameters, each pair comprising a wavelength λ and a corresponding modulation frequency fmod.

The light source 11a can be intensity-modulated by means of any known electrical or mechanical means.

The light irradiating the target 2 can be emitted continuously or in a pulsed manner by a given light source 11a.

The incident light on the target 2, emitted by the intensity modulated light source(s) 11a, propagates towards the target 2 and then through this target 2 (phenomenon symbolized by solid line arrows on Figure 1). The transmitted light is then gradually absorbed by the different constituents of the target 2, up to a characteristic depth called zmax which depends on the structure of the target 2 and on its physico-chemical composition.

The absorption of light energy causes local heating of the target 2. As a result, a thermal wave having a frequency equal to the modulation frequency fmod of the light source propagates in the target 2 (phenomenon symbolized by dotted line arrows on Figure 1), in particular towards the surface of the target 2.

This thermal wave gives rise in the fluid medium surrounding the target 2 to a pressure wave having the same frequency (that is to say a frequency equal to the modulation frequency fmod), which propagates in this fluid (for example gaseous) medium, and in particular in the detection cell 12 (phenomenon symbolized by alternating dotted arrows in Figure 1).

### Signal processing module 13

The non-invasive sensor 1 also comprises a signal processing module 13. The signal processing module 13 can comprise an analog-to-digital converter configured to convert a signal received from an acoustic transducer, such as an analog electrical signal, into a digital signal.

The signal processing module 13 can comprise a synchronous sensing device configured for demodulating and extracting a signal of interest from a signal detected and transmitted by an acoustic transducer.

The signal processing module 13 optionally comprises an operational amplifier operationally connected to the analog-to-digital converter and configured to amplify a digital signal derived from a signal transmitted by an acoustic transducer.

In some embodiments, the analog-to-digital converter can be functionally connected to a digital signal processor.

### Adaptation module 14

The non-invasive sensor 1 according to the invention further comprises an adaptation module 14 for adapting the irradiation parameters.

The adaptation module 14 is a computerized device comprising at least one processor:
- which can receive data from the detection cell 12 and/or from the signal processing module 13 if necessary;
- which can transmit data to the control device(s) 11c of at least one irradiation parameter of the light source 11a (for example a modulation frequency at which the intensity modulation device 11b modulates the intensity of a light emitted by the light source 11a, a wavenumber of a light emitted by the light source 11a, a power of the light source 11a, etc.).

### Photoacoustic detection cell 12

Figure 2 shows an exemplary embodiment of a photoacoustic detection cell 12.

The photoacoustic detection cell 12 comprises :
- a detection chamber 121 delimited by walls 123 and filled with a gas (e.g., air) through which acoustic waves can propagate,
- at least one acoustic transducer 122.

At least one of the walls 123 of the detection chamber, called contact wall 123a, is intended to be placed in contact with the target on part of or its whole external surface (external being meant relatively to the detection chamber) 2.

A contact wall 123a can be discontinuous in that it is formed in a solid material but can comprise at least one through hole 123a1.

The at least one through hole 123a1 allows the pressure wave to propagate from the target 2 up to at least one acoustic transducer 122 through the gas filling the detection chamber 121. Some of or all the walls 123 of the detection chamber can be continuous.

Some of or all the walls 123 of the detection chamber can be discontinuous due to one or more purposive through holes such as a vent.

The at least one acoustic transducer 122 is configured to convert the pressure of the acoustic wave into a signal that is exploitable by the signal processing module 13, such as an electrical signal. As non-limitative examples, an acoustic transducer 122 can be a microphone or a piezoelectric transducer.

Each electroacoustic sensor is functionally connected to the signal processing module 13.

The position(s) and/or the number of acoustic transducers 122 in the detection chamber can depend on the target and/or on the parameter(s) of interest and/or on embedding or miniaturization constraints.

In a particular embodiment, at least one acoustic transducer 122 is in contact with a wall 123 of the detection chamber.

In another embodiment, at least one acoustic transducer 122 is enshrined in a purposive through hole of a wall 123 of the detection chamber, for example a wall 123 different from the contact wall 123a, for example a wall 123 opposite to the contact wall 123a, as represented on figure 2.

In yet another embodiment, at least one acoustic transducer 122 can be placed outside the detection chamber 121 but in acoustic contact with the detection chamber 121, for example via an acoustic channel passing through a wall 123 of the detection chamber.

The geometrical parameters characterizing the detection chamber 121 comprise a reference internal volume Vref . The reference internal volume Vref is the volume of gas enclosed in the detection chamber 121 when the detection cell 12 is in contact at a predetermined pressure with a predetermined target 12 on the entirety of the external surface of the contact wall 123a, including if any the volume of the through hole 123a1.

When the detection cell 12 behaves as a closed detection cell, its behavior is well described by the so-called gas piston model developed by Rosencwaig and Gersho, and, as recalled in Kottmann et al., Mid-Infrared Photoacoustic Detection of Glucose in Human Skin: Towards Non-Invasive Diagnostics, Sensors (Basel). 2016 Oct 10;16(10):1663, the amplitude of the photoacoustic signal is inversely proportional to the reference volume Vref.

Figure 2 can represent a case where the photoacoustic detection sensor 1 is in its "reference position", which is the position associated with the reference internal volume Vref. In the case of figure 2, the contact between the contact wall 123a and the target 2 is optimal and allows the gas volume to be considered for the acoustic wave generated in the detection chamber 121 to be equal to the reference internal volume Vref. Therefore, the position of figure 2 is called "reference position".

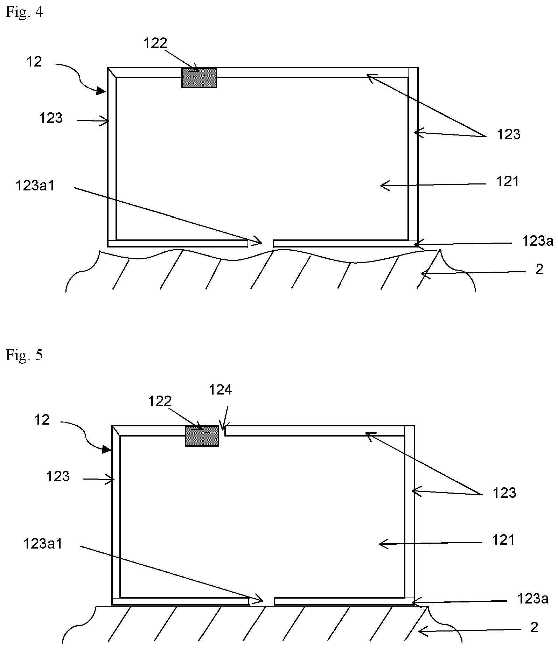

Figure 3 is a reproduction of figure 4C of EP3885765. This figure shows the amplitude of the pressure wave generated in a detection chamber having a reference volume equal to 5 µL and connected to the ambient air through a tube, the length of the tube being 9.4 mm, for various tube diameters, equal to 110 µm, 220 µm and 360 µm, respectively, for modulation frequencies of the irradiation light ranging from 10² Hz to 10⁴ Hz.

In the case of figure 3, the detection chamber can be modeled by a low pass filter regarding the modulation frequency of the irradiation light (and hence the frequency of the acoustic wave generated in the environment of the target 2) when the tube diameter is equal to 120 µm or 240 µm. In this case, the detection chamber acts as a closed detection chamber.

In contrast, the detection chamber of figure 3 can be modeled by a band pass filter having a resonance frequency of around 2.1 ^{∗} 10³ Hz when the tube diameter is equal to 360 µm. In this case, the detection chamber acts as an open detection chamber.

Most likely, for intermediate values of the tube diameter, higher than 240 µm and lower than 360 µm, the detection chamber behaves as a semi-closed cell.

EP3885765 aims at avoiding the open detection chamber 121 case by carefully selecting the geometric parameters of the detection chamber 121 at the design stage.

Even if this practice seems logical and reasonable, it is not sufficient in itself. Indeed, the inventors of the present application were confronted to the fact that, during the life of a given photoacoustic chamber, the detection chamber 121 can act among others as an acoustic low pass filter or an acoustic band pass filter despite all due care in the design of the detection cell 12 and in particular of the detection chamber 121.

Consequently, they have performed numerous experiments, including in silico experiments, in order to understand the phenomena that could lead to a change in the acoustic behavior of the detection chamber 121.

For example, extensive simulations were performed with COMSOL Multiphysics^{®} 6.1. The detection cell was modeled in 3 dimensions with a more or less fine mesh and with different models of leaks and its acoustic response was obtained in each case with COMSOL Multiphysics^{®}.

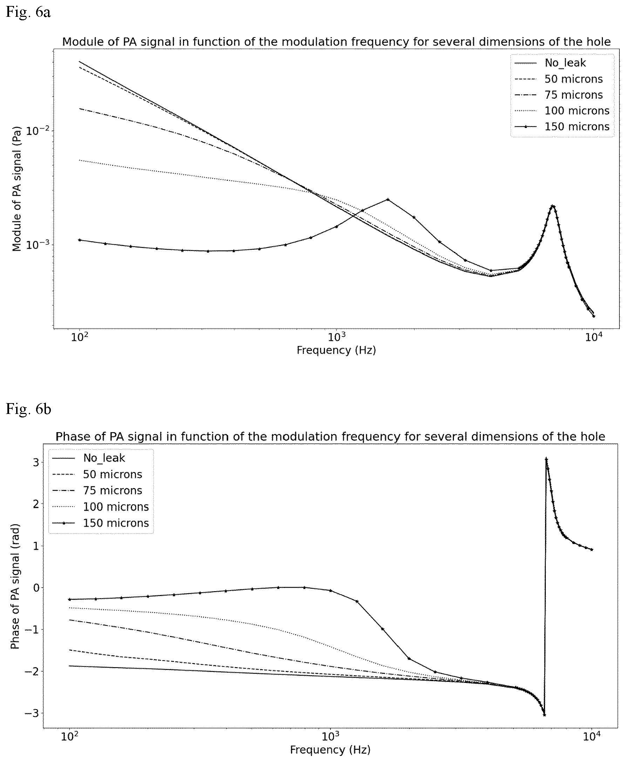

Figure 6a (respectively Figure 6b) shows such a simulated acoustic response in terms of amplitude (respectively phase) of the pressure wave of a model of detection chamber of a photoacoustic sensor, for several diameters (50 µm, 75 µm, 100 µm, 150 µm) of a cylindrical hole situated near the microphone and simulating acoustic leaks, compared to the response of the same detection chamber with no hole.

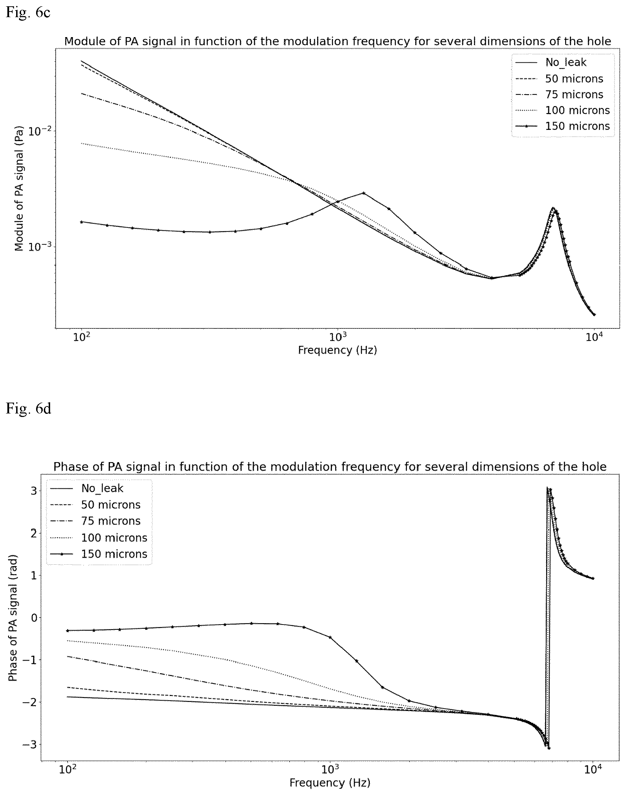

Figure 6c (respectively Figure 6d) shows such a simulated acoustic response in terms of amplitude (respectively phase) of the pressure wave of a model of detection chamber of a photoacoustic sensor, for several diameters (50 µm, 75 µm, 100 µm, 150 µm) of a cylindrical hole situated near a model of a skin target and simulating acoustic leaks, compared to the response of the same detection chamber with no hole.

Figures 6a, 6b, 6c and 6d show that:
- the acoustic response of the detection chamber can be more or less complex, such that in some cases, no analytic model can be fitted to this response, and
- when the size of the hole increases, the differences between the reference response (no leaks case) and the simulated acoustic response increase and the nature and/or the quantitative characteristics of the acoustic filter can change.

These experiments prove that the nature and the quantitative characteristics of the acoustic filter formed by the detection chamber 121 are not only determined by its initial geometric parameters as shown in EP3885765, but also can be associated with various changes in the geometric parameters characterizing the detection chamber 121 during its lifetime.

Among others, due to movements and/or deformations of the photoacoustic detection sensor 1 relative to the target 2, and/or to movements of parts of the components of the detection cell 12 relative to one another, it seems the volume of gas Vgas in which the acoustic wave is generated is not always the reference internal volume Vref but can be more or less than Vref.

A case where Vgas is higher than Vref is represented on figure 4 : in this case, due to variations of the pressure exerted on the target by the photoacoustic sensor 1, and/or to temperature and/or compositions variations in the target 2, and/or to a displacement of the contact wall 123a relative to the target 2, the contact with the target 2 is not maintained on the entire outer surface of the contact wall 123a and an additional volume is accessible to the gas in which the acoustic waves are generated, in addition to the reference internal volume.

The simulation of figures 6c and 6d could be for example representative of the leaks case represented on figure 4.

The experiments performed by the inventors, and in particular figure 6c and 6d, show that this additional volume can be sufficient to cause a change in the nature of the acoustic filter formed by the detection chamber 121 which can't be considered independently from this additional volume.

Such a change can be reversible in real conditions.

Note that the Rosencwaig-Gersho model can be no more relevant in such cases. The analytical relationship between Vref and the detected photoacoustic signal is so far not known but it is clear that other geometric parameters than Vref must be taken in account, among others the additional volume(s) the depth, and/or the external surfaces of these additional volume and their distribution in the detection cell 12.

Another cause of change in the behavior of the detection chamber 121 that has been observed by the inventors is the case where one or more components of the detection cell 12 that are enshrined in one or more walls 123 of the detection chamber 121 partially or totally unseal, for example due to excessive movements of the target 2 or aging of materials. This could be modeled by the apparition of a vent 124 or even an acoustic channel towards ambient air in the detection cell 12, as represented in figure 5. If the dimensions of this modeling vent or acoustic channel are sufficient, the behavior of the detection chamber 121 can switch from a closed cell, to a semi-closed cell or even to an open cell. Such a switch can be permanent or not.

The simulation of figures 6a and 6b could be for example representative of the leaks case represented on figure 5.

Last, the uncertainties in the assembly line of the detection cell 12 can be associated with unwished acoustic leaks from the beginning of the detection cell 12 despite the fact that it has been very carefully designed.

All these cases show the urgent need for a process for detecting acoustic leaks in a photoacoustic sensor 1 at any time of its lifetime, and in particular in situ that is to say while the photoacoustic sensor 1 is positioned relative to the target 2 to be analyzed.

### Process for detecting acoustic leaks

The process for detecting acoustic leaks in a non-invasive sensor 1 based on indirect photoacoustic detection and configured for measuring one or more parameters of interest in a target according to the invention comprises :
a) measuring with a detection cell 12 of the non-invasive sensor 1 a plurality of amplitudes and/or phases of pressure waves generated in the detection cell 12 in response to a plurality of irradiations of a test target with at least one light source 11a emitting a light characterized by at least one wavelength, the intensity of the respective emitted light being modulated at a modulation frequency that is different for each of the plurality of irradiations;
b) Determining with a processor at least one acoustic response of the detection cell 12 of the non-invasive sensor 1 as a function of a frequency of a pressure wave, based on the plurality of amplitudes and/or phases of pressure waves measured at a) and the respective modulation frequencies.
c) Comparing with a processor at least one determined acoustic response of the detection cell 12 to at least one reference acoustic response;
d) Based on the result of the comparison, providing with a processor a detection result characterizing the acoustic leaks in the detection cell 12.

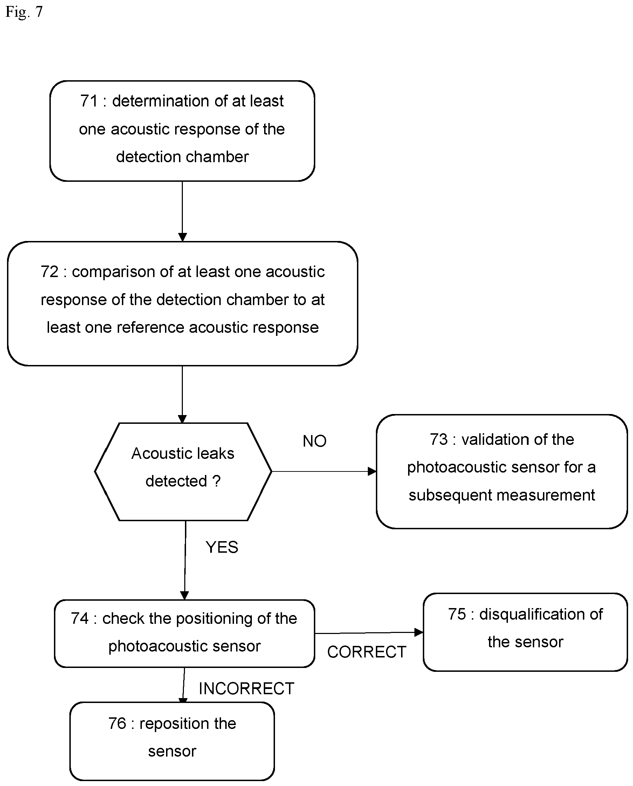

An exemplary embodiment of the process according to the invention is shown on figure 7. Determining an acoustic response of the detection cell 12 means in the context of the present document performing at least the minimal measurements required for obtaining a plurality of amplitudes and/or of phases of the pressure wave detected by the detection cell 12 for a plurality of modulation frequencies in given experimental conditions.

For example, the minimal measurements can allow to obtain a graph of the amplitude and/or a reference graph of the phase of the pressure wave detected by the detection cell 12 as a function of the modulation frequency of the irradiation light.

When the acoustic response can be associated with an analytical model, determining an acoustic response of the detection cell 12 can comprise determining at least the nature of the acoustic filter equivalent to the detection cell 12, for example if the detection cell 12 behaves as a low pass filter, a band pass filter, or any other type of filter regarding the modulation frequency of the irradiating light in the physico-chemical conditions in which the process is carried out.

To sum up, the determination of an acoustic response of the detection cell 12 can comprise the measurement of the amplitude and/or of the phase of the pressure wave generated in the detection chamber 121 of the detection cell 12 in response to an irradiation of a test target with the light source 11a at a given wavelength, for a plurality of modulation frequencies of the irradiation light in one or more ranges of modulation frequencies.

The number of modulation frequencies to be selected and their repartition in the one or more ranges of modulation frequencies can depend on the required precision and/or on an expected or an observed behavior of the detection cell 12 and/or on a total power consumption of the process.

The plurality of modulation frequencies can also be obtained with an automated modulation frequency sweep.

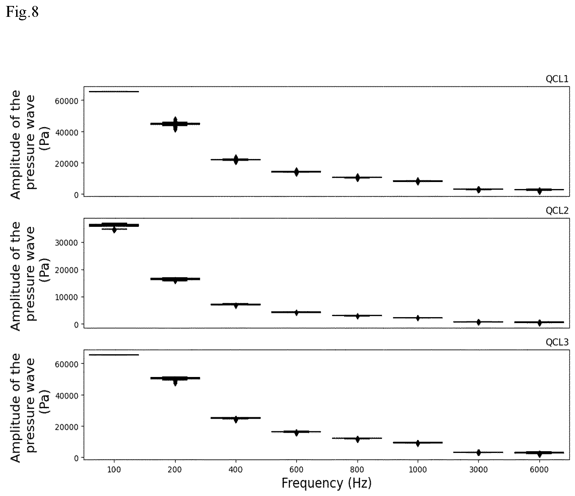

In a particular embodiment, several acoustic responses are determined for several wavelengths. The inventors have shown that, when the non-specific signal (for example due to absorption phenomena on the walls 123 of the detection chamber 121) can be neglected, the nature of the acoustic response of the detection chamber 121 in given experimental conditions doesn't depend on the wavelength of the irradiation light, as shown on figure 8 that was obtained with a first real non-invasive glucose sensor comprising three different light sources 11a: QCL1, having a wavenumber of 1034 cm⁻¹, QCL2, having a wavenumber of 950 cm⁻¹, and QCL3, having a wavenumber of 1125 cm⁻¹.

In this case, the nature of the acoustic response of the detection chamber 121 detected by the three QCLs is the same (low-pass filter) even if the quantitative characteristics (gain, cutoff frequency) of the acoustic responses are more or less different between the three QCLs.

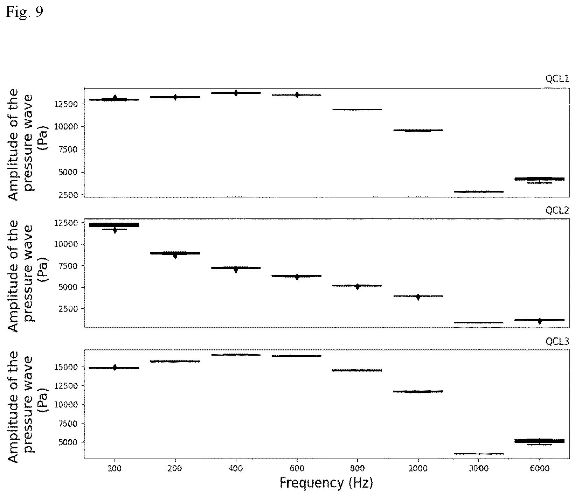

In a case such as the one of figure 8, the wavelength of the irradiation light can for example be the one that maximizes the mean intensity of the photoacoustic signal. Or a plurality of wavelengths can be implemented so as to reinforce the confidence level in the detection process. In some cases however, irradiations with different wavelengths can be associated with different determined acoustic responses for the same detection cell 12. This is the case of figure 9 that was obtained with a second real non-invasive glucose sensor comprising three different light sources 11a : QCL1, having a wavenumber of 1034 cm⁻¹, QCL2, having a wavenumber of 950 cm⁻¹, and QCL3, having a wavenumber of 1125 cm⁻¹.

In this case, the nature of the acoustic response of the detection chamber 121 detected by the three QCLs is the same (approximately band-pass filter) for QCL1 and QCL3 even if the quantitative characteristics (gain, cutoff frequency) of acoustic response slightly differ between these two QCLs, but the measurements of QCL 2 are associated with a low-pass filter. Inconsistencies between a plurality of determined acoustic responses of a given detection cell 12 can either be exploited in the subsequent comparison or lead to discarding some of the determined acoustic responses. For example, in the case of figure 9, the acoustic responses determined with QCL1 and QCL3 being consistent, the acoustic response determined with QCL2 could be discarded for the following steps of the process.

Once the at least one acoustic response of the detection cell 12 is determined, a processor, for example of the non-invasive sensor such as the processor of the adaptation module 14 or a dedicated processor, compares at least one of the determined acoustic responses of the detection cell 12 to at least one reference acoustic response.

The reference acoustic response can be determined theoretically.

For example, if the wished behavior is the one of a closed cell, the reference acoustic response could be a low pass filter. The processor determines in this case if the at least one reference acoustic response is a low pass filter, and optionally, if the acoustic response comprises one or more resonance frequencies.

The reference acoustic response can also be determined experimentally.

For example, determining the reference acoustic response can comprise the measurement of the amplitude and/or of the phase of the pressure wave generated in the detection chamber 121 of the detection cell 12 in response to an irradiation of a test target with the light source 11a at a given wavelength, for a plurality of modulation frequencies of the irradiation light in one or more ranges of modulation frequencies in reference conditions.

The number of modulation frequencies to be selected and their repartition in the one or more ranges of modulation frequencies can depend on the required precision and/or on an expected or an observed behavior of the detection cell 12 and/or on a total power consumption of the process.

The plurality of modulation frequencies can also be obtained with an automated modulation frequency sweep.

The reference conditions can comprise a reference position of the detection cell 12 relative to a reference target. The reference conditions can also comprise one or more environmental parameters, such as a temperature, an ambient pressure, a relative humidity, etc.

The result of the comparison between the at least one determined acoustic response of the detection cell 12 and the at least one reference acoustic response can be a Pearson correlation coefficient between the determined acoustic response and the reference acoustic response.

The result of the comparison between the at least one determined acoustic response of the detection cell 12 and the at least one reference acoustic response can also be a mean squared deviation of the determined acoustic response compared to the reference acoustic response or a spectral angle mapper (SAM) score.

The comparison between the at least one determined acoustic response of the detection cell 12 and the at least one reference acoustic response can also be based on a comparison metric involving one or more discrete or continuous parameters characterizing the determined or reference response, such as a number of resonance frequencies of the acoustic response of the detection cell 12, at least one sense of variation of the amplitude and/or the phase of the pressure wave generated in the detection cell 12 as a function of the frequency in at least one predetermined frequency range, a Pearson correlation coefficient, a mean squared deviation or a spectral angle mapper score, a weight being associated with each of the one or more parameters so as to provide a quantitative comparison score.

Table 1 hereafter shows the result of two comparison scores (spectral angle mapper (SAM), root-mean-square deviation (RMSD) or equivalently root-mean-square error (RMSE)) for the simulated acoustic responses of figure 6a compared to the simulated reference response (no leaks) of figure 6a.

**Table 1 :**

| Hole diameter | 50 µm | 75 µm | 100 µm | 150 µm |
|---|---|---|---|---|
| SAM | 0.03 | 0.29 | 0.61 | 1.16 |
| RMSE | 5,2×10⁻⁷ | 2,0×10⁻⁵ | 4,8×10⁻⁵ | 6,7×10⁻⁵ |

Table 2 hereafter shows the result of two comparison scores (spectral angle mapper (SAM), root-mean-square error (RMSE)) for the for simulated acoustic responses of figure 6c compared to the simulated reference response (no leaks) of figure 6c.

**Table 2 :**

| Hole diameter | 50 µm | 75 µm | 100 µm | 150 µm |
|---|---|---|---|---|
| SAM | 0.02 | 0.20 | 0.50 | 1.05 |
| RMSE | 2,3×10⁻⁷ | 1,1×10⁻⁵ | 4,0×10⁻⁵ | 6,4×10⁻⁵ |

Based on the result of the comparison, a processor, for example of the non-invasive sensor such as the processor of the adaptation module 14 or a dedicated processor, provides a detection result characterizing the acoustic leaks in the detection cell 12.

The detection result characterizing the acoustic leaks can be a binary result. For example, the result of the comparison can be compared with a predetermined threshold and if the comparison result is higher than the predetermined result, the detection result can be YES or 1, meaning that acoustic leaks have been detected. Otherwise, the detection result can be NO or 0, meaning that acoustic leaks have been detected.

For example, in the cases of Table 1 and Table 2, one can observe a change in the order of magnitude of both the SAM score and the RMSE score between a hole diameter of 50 µm and a diameter hole of 75 µm. By way of an example, the limit threshold could be in these cases set to 0.10 for the SAM score and of 1,0×10⁻⁶ for the RMSE.

The detection result characterizing the acoustic leaks can also be a continuous result. For example, the detection result can be the comparison score itself, or it can be an estimator of the importance of the acoustic leaks. In this case, sub-ranges of comparison score can be formed among the definition domain of the comparison score and each associated with a value characterizing the intensity of the acoustic leaks, for example from 0 to 10.

The detection result can be prompted on a user interface.

If the reference response is a low pass filter and a low pass filter without resonance frequency is observed, the result of the detection process can be that no acoustic leak has been detected. On the contrary, if a low pass filter with at least one resonance frequency is observed, the result of the detection process can be that a potential acoustic leak has been detected. If a band pass filter is observed, the result of the detection process can be that a significant acoustic leak has been detected.

Note that the example of a low-pass reference response has been described. Such a reference is adapted if low modulation frequencies are to be used for subsequent measurement of a parameter of interest in a main target 2 with the non-invasive sensor, for example if the main target 2 is to be analyzed in depth above its external surface.

In some cases, another reference response (and even a reference response that cannot be analytically modeled or that can be modeled piecewise) can be chosen. By way of an example, if the parameter of interest is to be measured near the external surface of the main target 2, a high pass response can be adapted.

Generally speaking, if the detection result is lower than a predetermined threshold, meaning that no or low enough acoustic leaks have been detected, the non-invasive sensor 1 can be allowed to perform at least one subsequent measurement of the parameter of interest in a main target 2. If the detection result is higher than a predetermined threshold, meaning that significant acoustic leaks have been detected, at least one instruction to remedy the detected acoustic leaks can be transmitted to the user of the non-invasive sensor 1.

The at least one instruction to remedy the detected acoustic leaks can comprise performing a checking of the position of the non-invasive sensor 1 relative to the test target.

In case the position of the non-invasive sensor 1 relative to the test target is checked by the user, if the user states that the position of the non-invasive sensor 1 relative to the test target is appropriate or if this position has already been checked at least once in a predetermined interval of time before this checking instruction, the user of the non-invasive sensor 1 can be further instructed to disqualify the non-invasive sensor 1 for subsequent measurements and optionally to replace the non-invasive sensor 1 with another one.

If the position of the non-invasive sensor 1 relative to the test target is incorrect, a user of the non-invasive sensor 1 can be further instructed to reposition the non-invasive sensor 1, after what steps a), b) and c) can be repeated.

In another embodiment, if the detection result is higher than a predetermined threshold, the non-invasive sensor 1 can optionally further either be allowed to perform at least one subsequent measurement and/or steps a), b) and c) can be repeated after a predetermined lapse of time in order to monitor the potential acoustic leak.

The invention also relates to a process for measuring at least one parameter of interest with a non-invasive sensor 1 based on indirect photoacoustic detection comprising carrying out the process for detecting acoustic leaks in the detection cell of a non-invasive sensor including a step e) on a given non-invasive sensor 1 with a given test target, and if the non-invasive sensor 1 is allowed at e) to perform at least one subsequent measurement, performing at least on subsequent measurement of the parameter of interest in a main target.

In a first embodiment, the test target for the process for detecting acoustic leaks is also the main target for a subsequent measurement of at least one parameter of interest with said non-invasive sensor. This allows in situ detection of acoustic leaks, for example periodical detection, while the non-invasive sensor 1 is installed on the main target. Such an embodiment is particularly suitable for a continuous glucose monitor, since it allows to reinforce the confidence level in the monitoring results.

In another embodiment, the test target for the process for detecting acoustic leaks is different from the main target for a subsequent measurement of at least one parameter of interest with the non-invasive sensor. This embodiment can be adapted for testing, and optionally qualifying a non-invasive sensor at the end of a production line.

In this last case, a test target can be designed in view of the detecting process. By way of nonlimiting examples, the test target can be made of polystyrene, graphite, poly(methyl methacrylate)) (PMMA) or poly(dimethylsiloxane) (PDMS).

The test target can have a smooth surface.

The test target can be soft or deformable.

The test target material can be chosen according to its thermal diffusivity.

The invention also relates to a computer program comprising instructions which, when the program is executed by a processor, cause the processor to carry out step b), step c) and step d) of the process for detecting acoustic leaks described above.

Last, the invention relates to a non-invasive sensor based on indirect photoacoustic detection comprising a detection cell 12, at least one light source 11a emitting a light characterized by at least one wavelength, the intensity of the respective emitted light being modulated at a modulation frequency that is tunable, and a processor configured for:
- determining at least one acoustic response of the detection cell 12 of the non-invasive sensor 1 as a function of a frequency of a pressure wave, based on a plurality of amplitudes and/or phases of pressure waves measured with the detection cell 12;
- comparing at least one determined acoustic response of the detection cell 12 to at least one reference acoustic response; and,
- based on the result of the comparison, providing a detection result characterizing acoustic leaks in the detection cell 12.

### LIST OF THE REFERENCE SIGNS

1: Non-invasive sensor based on indirect photoacoustic detection
11: Irradiation device
11a: light source
11b: device for modulating the intensity of the light source 11a
11c: device for controlling the modulation frequency fmod of the intensity of the light source
11a
12: photoacoustic detection cell
121 : detection chamber
122 : acoustic transducer
123 : wall of the detection chamber 121
123a : contact wall
123a1 : through hole in a contact wall
124: vent
13: Signal processing module
14: Adaptation module
2: Target

## Claims

1. Process for detecting acoustic leaks in a non-invasive sensor (1) based on indirect photoacoustic detection and configured for measuring at least one parameter of interest in a target, wherein said process comprises:
a) measuring with a detection cell (12) of the non-invasive sensor (1) a plurality of amplitudes and/or phases of pressure waves generated in the detection cell (12) in response to a plurality of irradiations of a test target with at least one light source (11a) emitting a light **characterized by** at least one wavelength, the intensity of the respective emitted light being modulated at a modulation frequency that is different for each of the plurality of irradiations;
b) Determining with a processor at least one acoustic response of the detection cell (12) of the non-invasive sensor (1) as a function of a frequency of a pressure wave, based on the plurality of amplitudes and/or phases of pressure waves measured at a) and the respective modulation frequencies.
c) Comparing with a processor at least one determined acoustic response of the detection cell (12) to at least one reference acoustic response;
d) Based on the result of the comparison, providing with a processor a detection result characterizing acoustic leaks in the detection cell (12).

2. Process according to claim 1 in which, at a), a plurality of acoustic responses of the detection cell (12) are determined with a plurality of acoustic transducers (122) and/or at a plurality of irradiation wavelengths, and at b) a plurality of determined acoustic responses is compared to a plurality of reference acoustic responses.

3. Process according to any of claims 1 to 2 in which the comparison is based on a comparison metric involving at least one metric variable chosen among a number of resonance frequencies of the acoustic response of the detection cell (12), at least one sense of variation of the amplitude and/or the phase of the pressure wave generated in the detection cell (12) as a function of the frequency in at least one predetermined frequency range, a spectral angle mapper score and a root-mean-square deviation.

4. Process according to any of claims 1 to 3 in which said detection result is a discrete variable.

5. Process according to any of claims 1 to 3 in which said detection result is a continuous variable.

6. Process according to any of claims 1 to 5 comprising after d):
e) if the detection result is lower than a predetermined threshold, allowing the non-invasive sensor (1) to perform at least one subsequent measurement of the parameter of interest in a main target and if the detection result is higher than a predetermined threshold, transmitting to a user of the non-invasive sensor (1) at least one instruction to remedy the detected acoustic leak.

7. Process according to claim 6 in which the at least one instruction is to check the position of the non-invasive sensor (1) relative to the test target.

8. Process according to claim 7 in which, if the user determines that the position of the non-invasive sensor (1) relative to the test target is correct, a user of the non-invasive sensor (1) is further instructed to disqualify the non-invasive sensor (1) for subsequent measurements and optionally to replace the non-invasive sensor (1).

9. Process for measuring at least one parameter of interest with a non-invasive sensor (1) based on indirect photoacoustic detection and configured for measuring at least one parameter of interest in a main target, the process comprising carrying out the process according to claim 6 on the non-invasive sensor (1) with a test target and if the non-invasive sensor (1) is allowed at e) to perform at least one subsequent measurement, performing at least one subsequent measurement of the at least one parameter of interest in said main target.

10. Process according to claim 9 wherein the test target for the process for detecting acoustic leaks is also the main target for a subsequent measurement of at least one parameter of interest with said non-invasive sensor (1).

11. Process according to claim 9 wherein the test target for the process for detecting acoustic leaks is different from the main target for a subsequent measurement of at least one parameter of interest with said non-invasive sensor (1).

12. Computer program comprising instructions which, when the program is executed by a processor, cause the processor to carry out step b), step c) and step d) of the process according to any of claims 1 to 11.

13. Non-invasive sensor based on indirect photoacoustic detection comprising a detection cell (12), at least one light source (11a) emitting a light **characterized by** at least one wavelength, the intensity of the respective emitted light being modulated at a modulation frequency that is tunable, and a processor configured for:
- determining at least one acoustic response of the detection cell (12) of the non-invasive sensor (1) as a function of a frequency of a pressure wave, based on a plurality of amplitudes and/or phases of pressure waves measured with the detection cell (12);
- comparing at least one determined acoustic response of the detection cell (12) to at least one reference acoustic response; and,
- based on the result of the comparison, providing a detection result characterizing acoustic leaks in the detection cell (12).
